# FASCICULE DE BREVET EUROPEEN

(11) **EP 2 363 169 B1**
(45) Date de publication et mention de la délivrance du brevet: **02.07.2014**
(21) Numéro de dépôt: 11156695.6
(22) Date de dépôt: 02.03.2011
(51) Int. Cl.: A61N 5/10, A61B 6/08, A61B 6/00, A61B 17/00

(54) **Objet-test et procédé utilisant cet objet-test permettant de contrôler la coïncidence des champs lumineux et des champs irradiés sur un appareil de traitement par radiothérapie**
Testobjekt und Verfahren zur Verwendung dieses Testobjekts, das die Kontrolle des Zusammenfalls der Lichtfelder und der bestrahlten Felder auf einem Strahlentherapiegerät ermöglicht
Test object and method using said test object suitable for checking the coincidence of illuminated fields and irradiated fields on a radiotherapy treatment apparatus

(30) Priorité: 05.03.2010 FR 1051639
(43) Date de publication de la demande: 07.09.2011
(73) Titulaire: Qualiformed, 85000 La Roche-sur-Yon (FR)
(72) Inventeur: Beaumont, Stéphane, 85430, Nieul le Dolent (FR); Villing, Margit, 85430, Nieul le Dolent (FR)
(74) Mandataire: Chaillot, Geneviève

(56) Documents cités:
- US-A1- 2002 114 426
- US-A1- 2003 185 348
- US-A1- 2006 002 519
- US-B1- 6 267 502
- LUCHKA K ET AL: "ASSESSING RADIATION AND LIGHT FIELD CONGRUENCE WITH A VIDEO BASED ELECTRONIC PORTAL IMAGING DEVICE", MEDICAL PHYSICS, AIP, MELVILLE, NY, US LNKD- DOI:10.1118/1.597867, vol. 23, no. 7, 1 juillet 1996 (1996-07-01), pages 1245-1252, XP000622096, ISSN: 0094-2405

## Description

La présente invention porte sur un objet-test et sur un procédé de contrôle de qualité d'un appareil de traitement par radiothérapie utilisant cet objet-test, et consistant à vérifier la coïncidence d'un faisceau de rayonnements ionisants de traitement et d'un faisceau lumineux de simulation, destiné à matérialiser ce faisceau de rayonnements ionisants. Dans ce qui suit, le faisceau de rayonnements ionisants de traitement par radiothérapie sera appelé faisceau d'irradiation et le faisceau lumineux destiné à matérialiser ce faisceau d'irradiation sera simplement appelé faisceau lumineux.

Dans un traitement par radiothérapie, on stérilise les tumeurs en les irradiant à haute dose à l'aide de multiples faisceaux de rayonnements ionisants (le plus souvent des rayons X, mais on utilise également d'autres rayonnement tels que des faisceaux d'électrons, de neutrons, d'ions) concentriques ou non. Le positionnement du patient par rapport aux faisceaux d'irradiation est primordial : en effet, un mauvais positionnement du patient par rapport aux faisceaux d'irradiation se traduira d'une part par une irradiation incomplète de la tumeur, ce qui peut induire une récidive de la maladie, et d'autre part par une irradiation des tissus sains avoisinant la tumeur, ce qui peut provoquer des graves complications pour le patient.

Pour garantir ce ciblage des faisceaux d'irradiation sur la tumeur, plusieurs tests de contrôle de qualité des appareils de traitement par radiothérapie ont été mis au point, lesquels sont effectués sur l'appareil de traitement par radiothérapie avant le traitement par radiothérapie d'un patient.

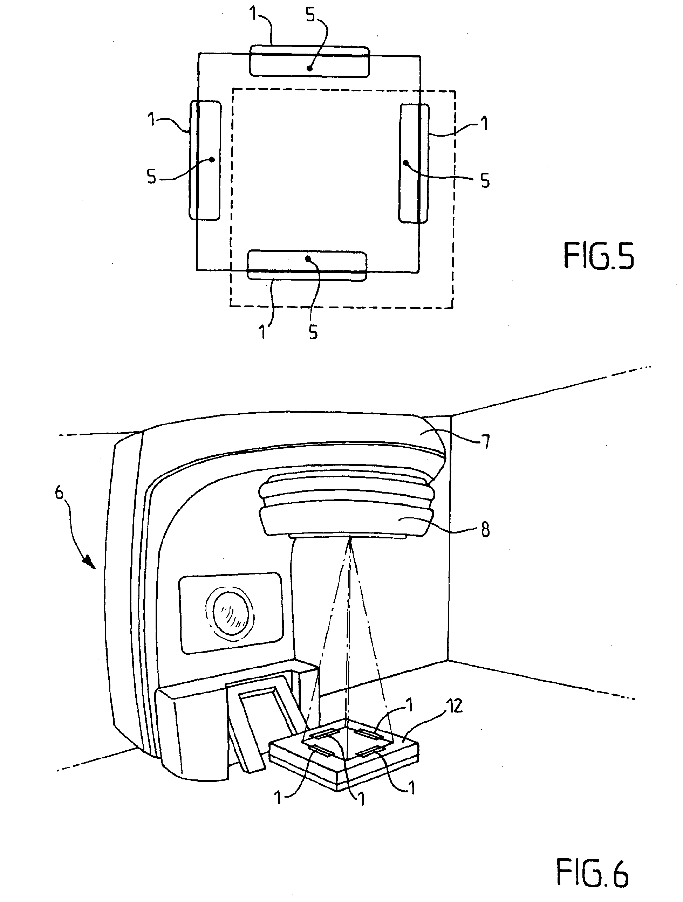

Un appareil de traitement par radiothérapie classique, représenté par exemple sur la Figure 6, comprend un statif tournant, portant à une extrémité une tête d'irradiation qui se termine par un collimateur qui permet de délimiter le faisceau d'irradiation et à l'autre extrémité un imageur appelé imageur portal qui permet de faire des radiographies numériques d'un objet placé entre le collimateur et l'imageur, en général sur une table de traitement, également appelée support patient, ou directement sur l'imageur.

La tête de l'appareil de traitement par radiothérapie projette en direction du support patient puis de l'imageur un faisceau d'irradiation destiné au traitement du patient, lequel passe à travers le collimateur qui est la partie finale de la tête et qui permet de délimiter le faisceau d'irradiation selon de nombreuses configurations. Parmi ces configurations, on retrouve pour de nombreux contrôles de qualité de ce collimateur un réglage de celui-ci pour délimiter des faisceaux de section rectangulaire.

Afin de pouvoir positionner correctement le patient sur le support patient par rapport au faisceau d'irradiation émis, lequel est invisible à l'oeil nu, le collimateur comprend également une source lumineuse, matérialisant le faisceau d'irradiation projeté à partir du collimateur. Le faisceau lumineux projeté à partir du collimateur a également une section rectangulaire, délimitée par le collimateur dans le cadre de nombreux contrôles de qualité du collimateur.

Parmi ces contrôles de qualité du collimateur de l'appareil de traitement par radiothérapie, l'un d'entre eux consiste à vérifier la coïncidence entre le faisceau d'irradiation et le faisceau lumineux permettant de le matérialiser. Ce contrôle est primordial puisque, dans une phase préalable, le patient est aligné sous l'appareil de traitement par radiothérapie au moyen de repères cutanés que l'on aligne en face du faisceau lumineux, pour être ensuite traité par le faisceau d'irradiation.

Pour un traitement efficace du patient positionné par rapport au faisceau lumineux, les bords du faisceau lumineux et du faisceau d'irradiation ne doivent pas être séparés de plus de 1 à 2 mm, tolérance maximale acceptée selon des recommandations nationales et internationales.

Le brevet américain US 6267502 B1 décrit un objet-test plat, portant un élément radioopaque.

L'objet-test de l'invention est destiné à faciliter le test de la coïncidence faisceau lumineux et faisceau irradié (faisceau de rayonnements ionisants de traitement) d'un appareil de traitement par radiothérapie, ledit appareil de traitement par radiothérapie comprenant un statif portant une tête d'irradiation, comprenant elle-même une source de rayonnements ionisants, une source lumineuse, et un collimateur, le collimateur délimitant à partir de la source de rayonnements ionisants un faisceau d'irradiation à section de préférence rectangulaire et délimitant à partir de la source lumineuse un faisceau lumineux de simulation à section de préférence rectangulaire, chaque bord du faisceau lumineux étant destiné à matérialiser de façon visible pour l'oeil humain un bord correspondant du faisceau d'irradiation, et des moyens de détection du faisceau d'irradiation vers lesquels est dirigé le faisceau d'irradiation et sur lesquels peut être disposé l'objet-test, lesdits moyens de détection du faisceau d'irradiation pouvant être constitués par un film radiosensible disposé sur un support patient placé sous le collimateur, ou par un imageur portal, porté par le statif et disposé à l'extrémité du statif opposée à celle portant le collimateur. L'objet-test peut également être disposé sur le support patient lorsque l'imageur portal est utilisé pour détecter le faisceau d'irradiation.

L'objet-test selon l'invention est défini dans la revendication indépendante 1, le procédé de contrôle de qualité d'un appareil de traitement par radiothérapie dans la revendication 12. Les revendications dépendantes renvoient aux caractéristiques particulières de l'invention.

La présente description a donc pour objet un objet-test pour un test de contrôle de qualité d'un appareil de traitement par radiothérapie, l'objet-test étant constitué d'un corps de densité électronique d2 et d'au moins un élément intégré sur une surface du corps ou noyé dans celui-ci et apte à être distingué du corps par imagerie par faisceaux de rayonnements ionisants, le corps comprenant en outre une surface de base sur laquelle il repose en utilisation et des moyens d'alignement sur un bord d'un faisceau lumineux de simulation émis par une source lumineuse située à l'intérieur de l'appareil de traitement par radiothérapie, ledit bord du faisceau lumineux simulant un bord d'un faisceau d'irradiation émis par l'appareil de traitement par radiothérapie pour traiter un patient, le corps étant destiné en utilisation à être placé de telle sorte que l'au moins un élément apte à être distingué du corps par imagerie se trouve dans le faisceau d'irradiation émis par l'appareil de traitement par radiothérapie, caractérisé par le fait que le corps est un corps mince et que lesdits moyens d'alignement sont espacés dudit ou desdits éléments, de telle sorte que, dans le plan de la surface de base du corps, la projection orthogonale des moyens d'alignement et la projection orthogonale dudit ou desdits éléments sont espacées d'une distance prédéterminée h.

Dans cette description, imagerie signifiera imagerie par faisceaux de rayonnements ionisants.

Le corps est mince, c'est-à-dire qu'il a une épaisseur faible par rapport à sa longueur et sa largeur. La détection du faisceau d'irradiation est ainsi très peu perturbée par la largeur de l'objet-test traversée. Le corps est de préférence rigide.

Le plan de la surface de base du corps de l'objet-test est dans un plan orthogonal à l'axe du faisceau lorsque l'objet-test est en utilisation pour un contrôle de qualité de l'appareil de traitement par radiothérapie.

La surface de base du corps est définie comme étant la surface sur laquelle repose le corps de l'objet-test lorsque celui-ci est en utilisation pour un contrôle de qualité de l'appareil de traitement par radiothérapie.

De préférence, la distance h est comprise entre 5 et 10 mm, et de manière davantage préférée, est égale à 8 mm.

L'au moins un élément apte à être distingué du corps par imagerie, qui peut être de densité électronique d1, peut être distingué du corps de densité électronique d2 par imagerie par faisceaux de rayonnements ionisants si, dans une image de l'objet-test par rayonnements ionisants tels que des rayons X, on peut distinguer cet au moins un élément du corps de densité électronique d2 sur l'image de l'objet-test obtenue.

Ainsi, lorsque l'on fait l'image avec le faisceau d'irradiation de l'objet-test positionné entre le collimateur et les moyens de détection des faisceaux d'irradiation, par exemple sur le support patient ou directement sur les moyens de détection des faisceaux d'irradiation, les moyens d'alignement de l'objet-test ayant été au préalable alignés sur les bords du faisceau lumineux émis par l'appareil de radiothérapie et la distance h entre le ou les éléments aptes à être distingués du corps par imagerie et les moyens d'alignement étant prédéterminée donc connue, on peut déterminer la distance entre le bord du faisceau d'irradiation observé sur l'image et le bord du faisceau lumineux, dont la position est déduite grâce à l'image de l'élément ou des éléments aptes à être distingués du corps par imagerie. L'objet-test est orienté de telle sorte que le ou les éléments aptes à être distingués du corps par imagerie sont à l'intérieur du faisceau lumineux et donc du faisceau d'irradiation.

La densité électronique d'un matériau est définie comme étant le nombre d'électrons contenus par centimètre-cube. Elle peut s'exprimer relativement à l'eau. Elle se calcule comme le produit de : Masse volumique du matériau (g/cm³) * Nombre d'Avogadro (6,0228 10²³ atomes/mole) * Z (Numéro atomique du matériau) / A (masse atomique du matériau).

Cette densité électronique est souvent exprimée relativement à celle de l'eau (densité électronique relative).

Dans ce qui suit, la densité électronique relative d'un matériau signifiera la densité électronique de ce matériau par rapport à celle de l'eau, ou densité électronique du matériau divisée par la densité électronique de l'eau (∼3,34 10²³ électrons/cm³).

Selon une caractéristique particulière, l'au moins un élément apte à être distingué du corps par imagerie a une densité électronique d1 différente de d2 et le rapport entre la densité électronique d1 et la densité électronique d2 est supérieur ou égal à 2, de préférence supérieur ou égal à 10.

Ainsi, le rapport de densité électronique entre l'élément de densité électronique d1 et le matériau de densité électronique d2 permet, lors de l'imagerie par un faisceau d'irradiation, de détecter la position de l'élément de densité électronique d1 dans le champ de rayonnement de radiothérapie avec un bon contraste.

Selon un premier mode de réalisation, l'au moins un élément de densité électronique d1 peut être une bille collée sur le corps de densité électronique d2 ou partiellement ou totalement noyée dans le corps de densité électronique d2, ou un fil collé sur ou incorporé dans le corps de densité électronique d2.

Ainsi, la bille de densité électronique d1 peut être noyée intégralement dans le matériau de densité électronique d2, ou peut être placée dans un trou percé dans le matériau de densité électronique d2, le trou ayant une profondeur égale à la moitié du diamètre de la bille, de telle sorte que la bille fait saillie de la surface de l'objet-test d'une hauteur égale à son rayon. On peut ainsi facilement repérer sa position par toucher.

Selon une autre caractéristique particulière, la bille de densité électronique d1 a un diamètre de 2 mm, afin de pouvoir être détectable, notamment à l'oeil nu ou par des moyens informatiques, sur une image d'un faisceau d'irradiation.

La forme de bille (ou d'un cylindre) est la forme préférée pour l'élément de densité électronique d1, car la forme sphérique de la surface assure que l'image par le faisceau d'irradiation de l'élément de densité électronique d1 détectée par les moyens de détection du rayonnement est la même, quelles que soient les conditions de divergence du faisceau d'irradiation et donc quelle que soit la largeur du faisceau d'irradiation.

L'au moins un élément de densité électronique d1 peut de manière plus générale avoir aussi n'importe quelle forme, et peut notamment par exemple être un cube, un fil, séparé d'une largeur fixe et prédéterminée donc connue des moyens d'alignement sur un bord du faisceau lumineux émis à partir de la source lumineuse.

L'élément de densité électronique d1 est évidemment destiné à être placé en utilisation à l'intérieur du champ lumineux qui représente le champ d'irradiation, donc à l'intérieur du champ d'irradiation.

Si l'élément de densité électronique d1 n'est pas dans le champ d'irradiation, son image ne peut pas être détectée sur les moyens de détection des faisceaux d'irradiation.

Selon encore une autre caractéristique particulière, le corps de densité électronique d2 peut être en polymère thermoplastique ou thermodurcissable ou en matériau composite, et l'au moins un élément de densité électronique d1 peut être en métal, de préférence en tungstène. Le polymère thermoplastique peut être par exemple un polycarbonate (PC) ou un polyméthacrylate de méthyle (PMMA) ou un polyoxyméthylène (POM) ou encore un polychlorure de vinyle (PVC) et le matériau composite peut être une feuille de fibres de carbone stratifiée avec une réside époxy.

Une couleur blanche (matière brute blanche ou peinture blanche) pour le corps de l'objet-test en matériau de densité électronique d2 facilitera la visualisation du faisceau lumineux et notamment de ses bords.

Selon un autre mode de réalisation, l'au moins un élément apte à être distingué du corps par imagerie est de l'air constitué d'un trou traversant, percé dans le corps de densité électronique d2. Le contraste dans l'image obtenue de l'objet-test entre le corps et le trou traversant permet de situer le trou sur l'image obtenue de l'objet-test.

Les moyens d'alignement sur un bord du faisceau lumineux peuvent être constitués par une ligne principale gravée ou imprimée sur au moins une partie d'une surface du corps de l'objet-test, la ligne principale ayant de préférence une largeur de 0,2 mm.

Facultativement, une peinture peut être déposée sur la ligne pour accentuer la perception visuelle de la ligne par un utilisateur de l'objet-test. Cette ligne peut notamment être noire pour accentuer le contraste avec le corps blanc et faciliter ainsi l'alignement.

L'objet-test peut avoir une forme rectangulaire, plate, de préférence d'épaisseur constante 2 mm.

L'objet-test peut également avoir une forme rectangulaire, plate, de préférence d'épaisseur principale 0,5 mm et comporter au moins deux éléments aptes à être distingués du corps par imagerie, de préférence des billes de diamètre de 2 mm, intégrés à des montants en saillie sur la surface du corps opposée à la surface de base, les montants ayant de préférence une hauteur de 2 mm à partir de la surface de base et facultativement une section carrée ou ronde de largeur 4 mm, les moyens d'alignement étant parallèles à un bord longitudinal de l'objet-test et à la ligne joignant les au moins deux éléments aptes à être distingués du corps par imagerie. Les montants peuvent, de préférence, être positionnés à deux coins d'un bord longitudinal de l'objet-test. Cette configuration de l'objet-test permet de réduire au maximum l'épaisseur du corps de densité électronique d2 dans la zone ne portant pas les éléments aptes à être distingués du corps par imagerie. La région entre les montants et les moyens d'alignement sur le faisceau lumineux étant destinée à recevoir la limite du faisceau d'irradiation (définie comme étant la zone du faisceau d'irradiation où l'intensité est égale à 50% de l'intensité maximale du rayonnement), l'épaisseur minimale du corps dans cette région permet d'éviter de perturber la détection de ce 50 % d'intensité du faisceau d'irradiation. Les montants plus épais, en dehors de cette région, permettent de supporter les billes.

L'objet-test, dans ce mode de réalisation, a de préférence deux éléments aptes à être distingués du corps par imagerie, mais pourrait également comporter un unique élément apte à être distingué du corps par imagerie, ou plus de deux éléments aptes à être distingués du corps par imagerie.

L'objet-test a donc une épaisseur minimale dans la zone destinée à recevoir la limite de faisceau d'irradiation pour rendre minimale la perturbation provoquée par l'épaisseur de l'objet-test lors de la détection de la limite de faisceau par les moyens de détection du rayonnement.

De préférence, l'objet-test a l'épaisseur minimale pour porter un moyen d'alignement et pour ne pas perturber la détection de la limite de faisceau par les moyens de détection des faisceaux d'irradiation. L'épaisseur de l'objet-test est de préférence uniforme, sauf sur les régions de l'objet-test sur lesquelles est ou sont disposés le ou les éléments aptes à être distingués du corps par imagerie, l'épaisseur des régions de l'objet-test sur lesquelles est ou sont disposés le ou les éléments aptes à être distingués du corps par imagerie étant minimale pour pouvoir supporter ces éléments.

Selon une caractéristique particulière , l'épaisseur du corps de l'objet-test dans sa zone destinée à détecter la limite de faisceau d'irradiation émis par l'appareil de radiothérapie est inférieure à l'épaisseur du corps de l'objet-test dans sa zone portant l'au moins un élément apte à être distingué du corps par imagerie. De façon davantage préférée, le corps de l'objet-test dans sa zone destinée à détecter la limite de faisceau d'irradiation émis par l'appareil de radiothérapie est une couche mince ou un film, le corps comprenant dans sa région destinée à porter l'au moins un élément apte à être distingué du corps par imagerie des moyens pour supporter et maintenir l'au moins un élément apte à être distingué du corps par imagerie.

Le fait d'avoir deux billes dans l'objet-test, permet de repérer la position d'une grande largeur (distance entre les deux billes) du faisceau lumineux en détectant ces deux billes dans le faisceau d'irradiation, et ainsi de pouvoir contrôler d'une part, que les bords adjacents du faisceau lumineux sont perpendiculaires et que les bords opposés du faisceau lumineux sont parallèles, et d'autre part, que le bord du faisceau lumineux soit parallèle au même bord de faisceau irradié.

De préférence, des trous circulaires de profondeur égale au rayon des billes sont formés sur la surface supérieure des montants pour recevoir les billes.

Selon des caractéristiques particulières de l'objet-test, l'objet-test a une largeur de 15 mm, une épaisseur de 2 mm au niveau du ou des deux montants et 0,5 mm ailleurs, une longueur choisie entre 15 mm, 30 mm, 80 mm, 180 mm ou 280 mm, là où les billes ont un diamètre de 2 mm. Lorsque l'objet-test comporte un seul élément de densité électronique d1, son centre est situé à 2 mm d'un premier bord longitudinal de l'objet-test, sur une ligne médiane longitudinale de l'objet-test, la ligne principale est disposée à 5 mm de l'autre bord longitudinal de l'objet-test, a une profondeur inférieure à 0,2 mm et une largeur de 0,2 mm. Lorsque l'objet-test a deux éléments de densité électronique d1, ceux-ci sont disposés sur des montants en saillie comme indiqué ci-dessus, lesdits montants étant formés à deux coins opposés d'un premier bord longitudinal de l'objet-test, les montants étant de forme en coupe horizontale sensiblement carrée ou ronde, de 4 mm de côté ou de diamètre, la ligne principale étant formée à 10 mm du premier bord longitudinal de l'objet-test lorsque l'objet-test a les mêmes dimensions que celles indiquées ci-dessus en référence au cas où il n'y a qu'un seul élément de densité électronique d1. La hauteur des montants par rapport à la surface de base de l'objet-test est de 1,5 mm, l'épaisseur du corps de l'objet-test étant égale, à l'exception des montants, à 0,5 mm.

Il est décrit également un objet-test, caractérisé par le fait qu'il est constitué d'un cadre rectangulaire, chaque bord du cadre portant ou étant constitué par un objet-test tel que défini ci-dessus, l'au moins un élément apte à être distingué du corps par imagerie pour chaque objet-test étant situé à l'intérieur du cadre.

Avantageusement, le cadre a sensiblement les mêmes dimensions que les dimensions des faisceaux lumineux et des faisceaux d'irradiation mesurées sur les moyens de détection des faisceaux d'irradiation.

L'objet-test peut comporter en outre un film mince coopérant avec la surface de base du corps, au moins une face du film permettant un positionnement/repositionnement aisé de l'objet-test sur les moyens de détection des faisceaux d'irradiation de l'appareil de traitement par radiothérapie (imageur portal ou film radiosensible ou similaires) notamment lorsque l'incidence du faisceau d'irradiation va de droite à gauche (faisceau horizontal) ou de bas en haut (faisceau vertical : source de rayonnements ionisants en bas et moyens de détection des faisceaux d'irradiation en haut).

L'au moins une face du film mince permettant un positionnement/repositionnement aisé de l'objet-test peut être la face du film en contact avec la surface de base de l'objet-test, ou la face du film en contact avec les moyens de détection des faisceaux d'irradiation (imageur portal ou film radiosensible ou similaires).

Le film mince peut être un film adhésif double face, au moins l'une des deux faces présentant un faible pouvoir adhésif permettant de positionner/repositionner l'objet-test. L'autre face du film peut facultativement présenter également un faible pouvoir adhésif.

Le film mince peut également être une couche de peinture aimantée, coopérant avec une couche aimantée sur l'appareil de radiothérapie (imageur portal ou film radiosensible ou similaires).

Dans le cadre du procédé de contrôle de qualité d'un appareil de radiothérapie, on pourra par exemple utiliser un jeu de quatre objets-test séparés, ou un cadre portant quatre objets-test ou constitué de quatre objets-test reliés entre eux, chacun des objets-test étant alignés sur chacun des quatre bords de faisceau lumineux de simulation de section rectangulaire. On peut ainsi contrôler la coïncidence des quatre bords d'un faisceau lumineux à section rectangulaire simultanément c'est-à-dire sur la même image obtenue à partir des moyens de détection des faisceaux d'irradiation.

Il est décrit également un procédé de contrôle de qualité d'un appareil de traitement par radiothérapie, caractérisé par le fait qu'il comprend les étapes de :
a) positionnement de quatre objets-test tels que définis ci-dessus, sur des moyens de détection des faisceaux d'irradiation ou sur tout autre support interposé entre ces moyens de détection des faisceaux d'irradiation et le collimateur de l'appareil de traitement par radiothérapie avant ou après alignement des moyens d'alignement des quatre objets-test sur chacun des quatre bords d'un faisceau lumineux de simulation destiné à matérialiser le faisceau d'irradiation émis par l'appareil de traitement par radiothérapie, le ou les éléments aptes à être distingués du corps par imagerie de chacun des quatre objets-test étant placés à l'intérieur du faisceau lumineux ;
b) imagerie des objets-test par le faisceau d'irradiation détecté à l'aide des moyens de détection des faisceaux d'irradiation ;
c) calcul sur l'image obtenue, pour chaque objet-test, de la distance entre le ou les éléments aptes à être distingués du corps par imagerie et le bord du faisceau d'irradiation ;
d) déduction, pour chaque bord du faisceau d'irradiation, en fonction de la distance prédéterminée h entre le ou les éléments et les moyens d'alignement de la distance entre le bord du faisceau d'irradiation du bord du faisceau lumineux de simulation ;
e) facultativement, lorsque chaque objet-test présente au moins deux éléments aptes à être distingués du corps par imagerie, déduction à partir de l'image obtenue de l'orthogonalité et du parallélisme des bords de faisceau lumineux de simulation ou/et du parallélisme des bords de faisceau lumineux par rapport aux bords de faisceau d'irradiation ;
f) correction de la position de la source lumineuse, afin de faire correspondre chaque bord du faisceau lumineux avec chaque bord correspondant du faisceau d'irradiation ; et
g) répétition facultative des étapes précédentes jusqu'à ce que la distance entre les bords du faisceau d'irradiation et les bords du faisceau lumineux soit inférieure ou égale à une valeur seuil, et/ou jusqu'à ce que les deux bords adjacents des faisceaux soient perpendiculaires et que les deux bords opposés des faisceaux soient parallèles.

Le support interposé entre le collimateur et les moyens de détection des faisceaux d'irradiation peut notamment être le support patient ou le capot de l'imageur portal.

Lorsque les moyens de détection des faisceaux d'irradiation sont constitués par un imageur portal, les étapes d) à f) ci-dessus peuvent avantageusement être mises en oeuvre par un logiciel sur un ordinateur relié à l'imageur portal.

Pour mieux illustrer l'objet de la présente invention, on va en décrire plus en détail ci-après un mode de réalisation particulier, avec référence au dessin annexé.

Sur ce dessin :
- la Figure 1 est une vue de dessus schématique d'un objet-test selon un premier mode de réalisation de l'invention ;
- la Figure 2 est une vue frontale de l'objet-test de la Figure 1 ;
- la Figure 3 est une vue schématique de face d'un appareil de traitement par radiothérapie, sur lequel est disposé un objet-test selon la présente invention ;
- la Figure 4 est une vue de détail de la Figure 3, représentant une vue latérale de l'objet-test de la Figure 1 ;
- la Figure 5 est une vue schématique de dessus d'un jeu de quatre objets-test selon la présente invention, chacun étant aligné sur l'un des bords d'un faisceau lumineux à section transversale rectangulaire, les objets-test formant un cadre ;
- la Figure 6 est une vue en perspective de quatre objets-test selon l'invention, placés en utilisation directement sur l'imageur portal d'un appareil de traitement par radiothérapie pour un test de contrôle de qualité de celui-ci ;
- la Figure 7 est une vue analogue à la Figure 1 d'un objet-test selon un deuxième mode de réalisation de l'invention ;
- la Figure 8 est une vue analogue à la Figure 2 de l'objet-test selon le deuxième mode de réalisation de l'invention ;
- la Figure 9 est une vue analogue à la Figure 4 de l'objet-test selon le deuxième mode de réalisation de l'invention ;
- la Figure 10 est une vue analogue à la Figure 5, avec des objets-test selon le deuxième mode de réalisation de l'invention ;
- la Figure 11 est un schéma illustrant le positionnement de l'objet-test en fonction de l'intensité du rayonnement émis qui est détectée par les moyens de détection du faisceau d'irradiation ; et
- la Figure 12 est une vue de profil d'un objet-test selon le deuxième mode de réalisation de la présente invention, positionné sur un élément de type film adhésif repositionnable permettant le positionnement aisé de l'objet-test sur l'appareil de traitement par radiothérapie.

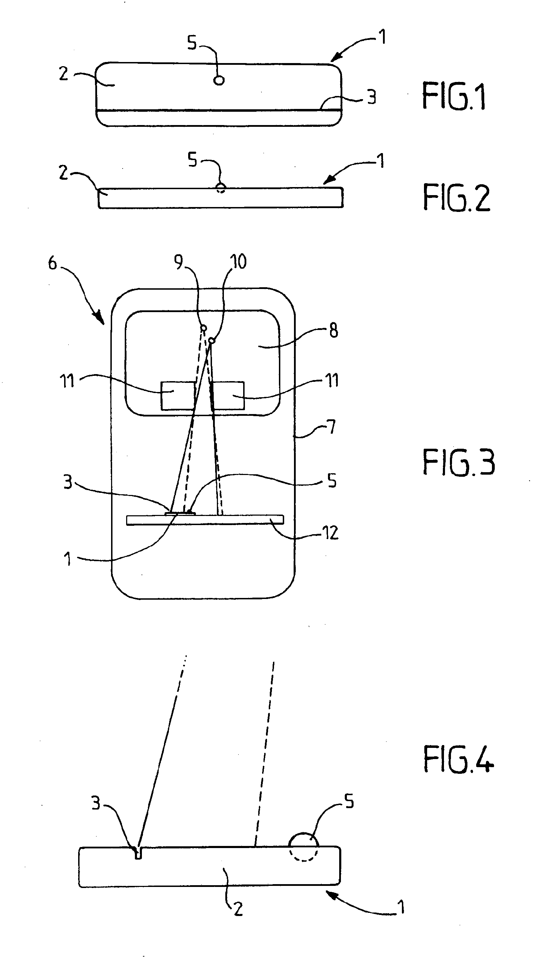

Si l'on se réfère aux Figures 1 et 2, on peut voir qu'il y est représenté un objet-test 1 selon un premier mode de réalisation , formé d'un corps rectangulaire mince et plat 2, à bords arrondis. Le corps 2 est dans le premier mode de réalisation préféré en polyméthacrylate de méthyle (PMMA) de couleur blanche et dans le deuxième mode de réalisation préféré en polychlorure de vinyle (PVC) de couleur blanche. Selon des caractéristiques préférées de l'objet-test selon la présente invention, le corps 2 a une épaisseur de 2 mm, une largeur de 15 mm, et une longueur choisie entre les valeurs de 15 mm, 30 mm, 80 mm, 180 mm ou 280 mm, selon les dimensions du faisceau d'irradiation issus de l'appareil de traitement par radiothérapie faisant l'objet du test de contrôle de qualité.

D'autres dimensions du corps 2 peuvent également être considérées, selon les caractéristiques de l'appareil de traitement par radiothérapie.

Une ligne continue 3 est formée au voisinage d'un bord longitudinal du corps 2. La ligne continue 3 est formée par gravure d'une rainure sur la surface du corps 2, ou par impression d'une ligne sur la surface du corps 2. Lorsque le corps 2 a les dimensions préférées mentionnées ci-dessus, la ligne continue 3 est formée à 5 mm d'un des bords longitudinaux du corps 2. La ligne continue 3 a une largeur d'environ 0,2 mm, et peut être formée par gravure de la surface du corps 2, sur une profondeur inférieure ou égale à 0,2 mm. Une peinture de couleur noire est de préférence appliquée sur la gravure, afin de faire ressortir la ligne continue 3 à la surface du corps 2.

Une bille en tungstène 5 est disposée sur le corps 2, par collage de celle-ci dans un trou formé sur une ligne médiane dans le sens longitudinal du corps 2, au voisinage du bord longitudinal opposé à celui où est formée la ligne continue 3 du corps 2, à une distance fixe prédéterminée h de la ligne continue 3. Le trou dans lequel est placée la bille 5 a une profondeur et un rayon égaux au rayon de la bille 5. Lorsque le corps 2 a les dimensions préférées mentionnées ci-dessus, la bille 5 a un diamètre de 2 mm, et le trou formé à la surface du corps 2 a une profondeur et un rayon de 1 mm. Le centre du trou est situé à 2 mm du bord longitudinal opposé au bord longitudinal du corps 2 au voisinage duquel est formée la ligne continue 3.

Le procédé de fabrication de l'objet-test selon le premier mode de réalisation comprend les étapes suivantes :
- usinage d'un bloc polymère thermoplastique pour former le corps 2 ;
- formation de la ligne 3 par gravure sur la surface du corps 2 ;
- facultativement dépôt d'une couche de peinture noire à l'intérieur de la gravure 3 ;
- perçage d'un trou de profondeur égale au rayon de la bille 5 au centre du corps 2, à une distance prédéterminée de la ligne 3 ;
- collage de la bille 5 dans le trou, de telle sorte que celle-ci fait saillie de la surface de l'objet-test d'une hauteur égale à son rayon ;
- facultativement finition par polissage des surfaces de l'objet-test.

Les bords et les tranches de l'objet-test 1 peuvent en outre facultativement être arrondis pour éviter à un opérateur de se couper lors de l'utilisation de l'objet-test.

Dans une variante de l'objet-test selon le premier mode de réalisation , le corps 2 est en métal atténuant peu les rayonnements ionisants, par exemple de l'aluminium, et de mêmes dimensions que dans la première variante à l'exception de l'épaisseur réduite au minimum, 1 mm pour de l'aluminium. La ligne 3 est gravée ou imprimée à la surface du corps 2. Afin d'obtenir un contraste de radio-opacité similaire à celui de la première variante entre le corps 2 en matière plastique et la bille en métal du mode de réalisation précédent, la bille est remplacée par un trou traversant le corps 2, formé sur le corps 2 au même emplacement que la bille dans la première variante, et de même diamètre que la bille dans la première variante.

Si l'on se réfère maintenant aux Figures 3 et 4, on peut voir qu'il y est représenté un objet-test selon le premier mode de réalisation de l'invention, en utilisation sur un appareil de traitement par radiothérapie.

L'appareil de traitement par radiothérapie 6 comprend un statif 7, portant une tête d'irradiation 8. Une source de rayonnements ionisants de radiothérapie 9, une source lumineuse de simulation 10 et un collimateur 11 sont montés à l'intérieur de la tête d'irradiation 8.

Le faisceau d'irradiation provenant de la source de rayonnements ionisants 9 (en pointillés sur les Figures 3 à 5) et le faisceau de la source lumineuse 10 (en ligne continue sur les Figures 3 à 5) sont délimités par le collimateur 11, de telle sorte qu'après le passage dans le collimateur 11, les faisceaux de la source de rayonnements ionisants 9 et de la source lumineuse de simulation 10 sont à section transversale rectangulaire, et sensiblement de mêmes dimensions, chaque bord du faisceau lumineux représentant une mâchoire du collimateur 11.

Un détecteur de rayonnements ionisants, dans le mode de réalisation représenté, un imageur portal 12, est placé sous la tête d'irradiation 8.

Le détecteur permet d'enregistrer l'image par le faisceau d'irradiation d'un objet placé entre le détecteur et le collimateur.

Un film radiosensible placé sur un support patient, ou tout autre détecteur de rayonnement, pourrait également être utilisé en tant que détecteur de rayonnement dans le cadre de la présente invention.

Le procédé de contrôle de qualité de l'appareil de traitement par radiothérapie va maintenant être décrit plus en détail, en rapport avec les Figures 3 à 6. Ce procédé de contrôle de qualité utilise un jeu de deux paires d'objets-test, les longueurs pour chaque paire d'objets-test étant choisies en fonction des dimensions du faisceau d'irradiation rectangulaire à contrôler. Il est à noter qu'un cadre, dont chaque côté serait constitué par un objet-test selon la présente invention, et de dimensions choisies en fonction des dimensions du faisceau d'irradiation de l'appareil de traitement par radiothérapie, pourrait également être utilisé.

Un premier objet-test 1 est placé sur l'imageur portal 12 (ou sur le moyen de détection des faisceaux d'irradiation ou sur le support patient), la source lumineuse 10 dans la tête d'irradiation étant allumée, et l'objet-test 1 est aligné de telle sorte que la ligne continue 3 de l'objet-test est alignée avec un premier bord du faisceau lumineux émis depuis la source lumineuse 10 à travers le collimateur 11, et de telle sorte que la bille 5 de l'objet-test est à l'intérieur des bords du faisceau lumineux et donc du faisceau d'irradiation émis, comme cela est représenté sur les Figures 3 à 5.

On place ensuite de manière similaire, selon une variante préférée du procédé de contrôle de qualité, trois autres objets-test sur l'imageur portal 12 sur les trois autres bords du faisceau lumineux, comme cela est représenté plus précisément sur les Figures 5 et 6.

On active ensuite la source de rayonnements ionisants 9, et on fait l'image des quatre objets-test par le faisceau d'irradiation sur l'imageur portal 12.

Les billes 5 (respectivement les trous pour la deuxième variante du premier mode de réalisation de l'objet-test) des objets-test 1, de densité électronique supérieure (respectivement inférieure) à la densité électronique du matériau formant le corps 2 de l'objet-test 1, sont visibles (respectivement relativement au corps) sur l'image des objets-test formée sur l'imageur portal.

Le bord du faisceau d'irradiation (défini comme étant généralement l'isosignal correspondant à entre 30 et 50 % du signal maximum, c'est-à-dire du signal à l'intérieur du faisceau d'irradiation) étant également visible sur l'image détectée de l'imageur portal 12, on peut détecter sur l'image, pour chaque bord du faisceau d'irradiation, la distance entre l'image du bord du faisceau d'irradiation et l'image de la bille 5 de l'objet-test placé sur le bord du faisceau lumineux correspondant.

L'objet-test ayant été aligné sur le bord du faisceau lumineux, la distance h entre la ligne continue 3 et la bille 5 sur l'objet-test 1 étant prédéterminée donc connue, et la distance entre l'image de la bille 5 et l'image du bord du faisceau d'irradiation étant mesurée, on peut déduire la distance entre le bord du faisceau lumineux et le bord du faisceau d'irradiation correspondant. On peut ainsi corriger la position du faisceau lumineux de simulation, afin que les quatre bords de celui-ci correspondent respectivement aux quatre bords du faisceau d'irradiation. Les étapes précédentes peuvent être répétées jusqu'à ce que les bords des deux faisceaux (faisceau d'irradiation et faisceau lumineux de simulation) soient alignés aux tolérances admises près, garantissant ainsi un bon positionnement du patient dans le faisceau d'irradiation par l'intermédiaire du positionnement du patient dans le faisceau lumineux de simulation.

Les étapes du procédé de contrôle de qualité de l'appareil de traitement par radiothérapie avec l'objet-test du premier mode de réalisation sont donc les suivantes :
- positionnement de quatre objets-test sur des moyens de détection des faisceaux d'irradiation (imageur portal 12) ;
- alignement des moyens d'alignement (lignes 3) des quatre objets-test sur chacun des quatre bords d'un faisceau lumineux de simulation destiné à matérialiser le faisceau d'irradiation, les éléments (billes 5 ou trous selon la variante) de chaque objet-test présentant une densité électronique d1 différente de la densité électronique d2 du corps de l'objet-test étant placés à l'intérieur du faisceau lumineux donc du faisceau d'irradiation;
- imagerie des objets-test par le faisceau d'irradiation ;
- calcul sur l'image obtenue, pour chaque objet-test, de la distance entre l'élément de densité électronique d1 et le bord du faisceau d'irradiation ;
- déduction, pour chaque bord du faisceau d'irradiation, de la distance par rapport au bord correspondant du faisceau lumineux ;
- correction de la position de la source lumineuse, afin de faire correspondre chaque bord du faisceau lumineux avec chaque bord du faisceau d'irradiation correspondant ;
- répétition facultative des étapes précédentes jusqu'à ce que la distance entre les bords du faisceau d'irradiation et les bords du faisceau lumineux de simulation soit inférieure ou égale à une valeur seuil, de préférence 1 mm.

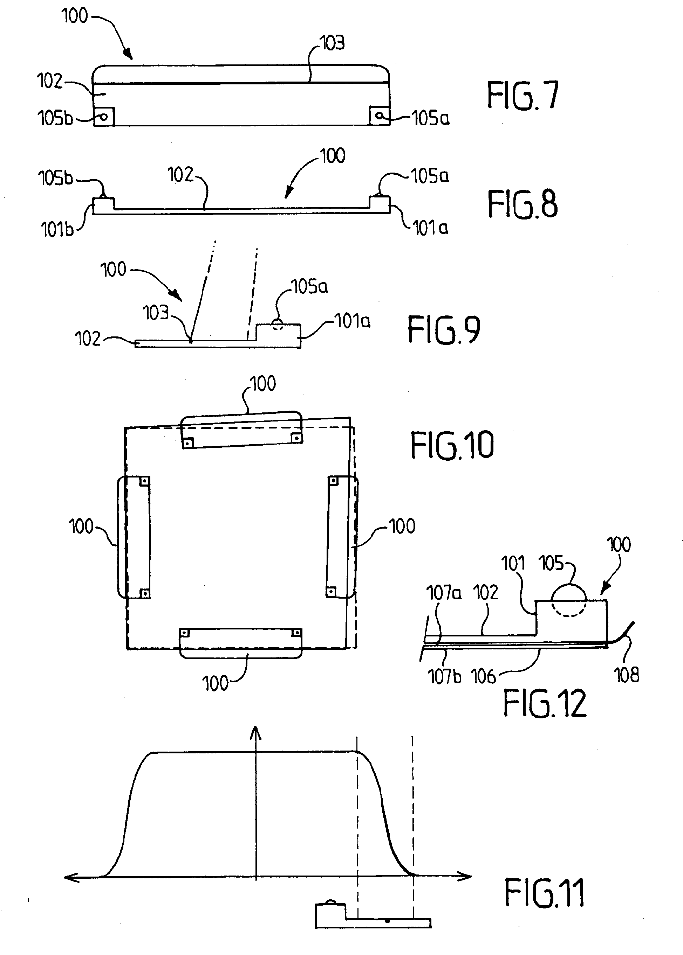

Si l'on se réfère aux Figures 7 à 9, on peut voir qu'il y est représenté un objet-test 100 selon un deuxième mode de réalisation.

L'objet-test 100 est constitué d'un corps mince et plat 102 de forme rectangulaire à bords arrondis, et de deux montants 101a et 101b, de forme allongée à base carrée, formés respectivement à deux coins d'un même bord longitudinal du corps 102, en saillie par rapport à la surface du corps 102. L'objet-test 100 porte, comme pour l'objet-test 1, une ligne 103 au voisinage du bord longitudinal opposé à celui sur lequel sont formés les montants 101a, 101b, sur la même surface du corps 102 que la surface sur laquelle sont formés les montants 101a, 101b. Les montants 101a, 101b et le corps 102 sont dans le mode de réalisation préféré en polychlorure de vinyle (PVC) de couleur blanche. Selon des caractéristiques préférées de l'objet-test selon la présente invention, le corps 102 a une épaisseur de 0,5 mm, une largeur de 15 mm, et une longueur choisie entre les valeurs de 15 mm, 30 mm, 80 mm, 180 mm ou 280 mm, selon les dimensions du faisceau rectangulaire d'irradiation de l'appareil de traitement par radiothérapie faisant l'objet du test de contrôle de qualité. Les montants 101a, 101b ont quant à eux, selon des caractéristiques préférées de l'objet-test, une hauteur de 1,5 mm, et une longueur et une largeur égales à 4 mm.

D'autres dimensions du corps 102 peuvent également être considérées, selon les caractéristiques de l'appareil de traitement par radiothérapie.

La ligne continue 103 est formée de manière analogue à la ligne 3 pour l'objet-test 1. Lorsque le corps 102 a les dimensions préférées mentionnées ci-dessus, la ligne continue 103 est formée à 5 mm d'un des bords longitudinaux du corps 102. La ligne continue 103 a une largeur d'environ 0,2 mm, et peut être formée par gravure de la surface du corps 2, sur une profondeur inférieure ou égale à 0,2 mm. Une peinture de couleur noire est de préférence appliquée sur la gravure, afin de faire ressortir la ligne continue 103 à la surface du corps 102.

Deux billes identiques en tungstène 105a et 105b sont disposées respectivement dans des trous formés sur la surface supérieure des montants 101a, 101b. Les trous dans lesquels sont placées les billes 105a et 105b ont une profondeur et un rayon égaux au rayon des billes 105a et 105b. Lorsque le corps 102 a les dimensions préférées mentionnées ci-dessus, les billes 105a et 105b ont un diamètre de 2 mm, et le trou formé centré sur la surface du corps 102 a une profondeur de 1 mm.

De manière analogue au premier mode de réalisation, une deuxième variante d'un objet-test selon le deuxième mode de réalisation est constitué par un corps 102 métallique léger, de préférence en aluminium d'épaisseur 0,5 mm, avec des montants 101a et 101b en aluminium, de préférence de 1,5 mm de hauteur et de section de 4 x 4 mm², au centre desquels sont formés des trous traversants dans le sens de la hauteur du montant, à la place de billes de la première variante pour constituer avec le corps un rapport de densité électronique remarquable sur une image par rayonnements ionisants (image du faisceau d'irradiation). Une autre variante plus rigide peut être constituée d'un corps 102 en polychlorure de vinyle (PVC) de couleur blanche de 0,5 mm d'épaisseur sur lequel sont fixés des montants 101a et 101b en aluminium, de préférence de 1,5 mm de hauteur et de section de 4 x 4 mm², au centre desquels sont formés des trous traversants dans le sens de la hauteur du montant, ces trous traversant également le corps en PVC.

Le procédé de fabrication de l'objet-test selon la première variante du deuxième mode de réalisation comprend les étapes suivantes :
- usinage d'un bloc de polymère thermoplastique pour former le corps 102 avec les deux montants 101a, 101b en saillie ;
- formation de la ligne 103 par gravure sur la surface du corps 102 ;
- facultativement dépôt d'une couche de peinture noire à l'intérieur de la gravure 103 ;
- perçage de trous de profondeurs égales au rayon des billes 105a et 105b au centre des montants 101a, 101b ;
- collage des billes 105a et 105b dans les trous, de telle sorte que celles-ci font saillie de la surface des montants 101a, 101b d'une hauteur égale à leur rayon ; et
- facultativement finition par polissage des surfaces de l'objet-test.

L'objet-test 100 s'utilise de manière analogue à l'objet-test 1, comme cela est représenté sur les Figures 3 et 6. La présence de deux billes 105a et 105b sur chaque objet-test 100 permet en outre de vérifier la perpendicularité de deux mâchoires contiguës du collimateur 11, et le parallélisme de deux mâchoires opposées du collimateur 11.

De manière analogue à ce qui a été décrit en référence à la Figure 5, avec les mêmes conventions de représentation pour le faisceau d'irradiation et le faisceau lumineux de simulation, on aligne les lignes 103 avec les bords du faisceau lumineux, et on fait l'image de l'objet-test par le faisceau d'irradiation. Les deux billes pour chaque objet-test permettent de repérer la position de chaque bord du faisceau d'irradiation par rapport au bord du faisceau lumineux, et ainsi de pouvoir corriger la position de la source lumineuse et/ou de pièces complémentaires aux mâchoires du collimateur 11 et/ou directement des mâchoires du collimateur 11 pour faire en sorte que les bords adjacents du faisceau d'irradiation ou/et lumineux sont perpendiculaires et que les bords opposés du faisceau d'irradiation ou/et lumineux sont parallèles.

Les étapes du procédé de contrôle de qualité de l'appareil de traitement par radiothérapie avec l'objet-test du second mode de réalisation sont identiques à celles pour le premier mode de réalisation, avec l'étape supplémentaire consistant à vérifier que les bords adjacents du faisceau d'irradiation ou/et lumineux soient perpendiculaires et que les bords opposés du faisceau d'irradiation ou/et lumineux soient parallèles, comme indiqué ci-dessus.

La Figure 11 représente un profil d'intensité du faisceau d'irradiation mesuré selon une médiane du champ d'irradiation de section rectangulaire, et le positionnement de l'objet-test dans ce champ d'irradiation. La limite du faisceau d'irradiation correspond à 50% de l'intensité maximale du faisceau d'irradiation, représentée sur la Figure 11 par le palier horizontal supérieur de la courbe. L'objet-test doit être placé dans le faisceau de telle sorte que l'épaisseur minimale de l'objet-test se situe au-delà de la limite de faisceau, et de telle sorte que les montants 101a et 101b portant les billes 105a et 105b se trouvent dans la zone d'intensité du faisceau d'irradiation, pour ainsi éviter de perturber, par une épaisseur de l'objet-test trop importante, la détection de cette limite du faisceau d'irradiation. On s'assure par ce positionnement que la limite de faisceau d'irradiation n'est pas perturbée par l'épaisseur de l'objet-test au niveau de la limite de faisceau. L'objet-test a ainsi une épaisseur minimale dans la zone destinée à détecter la limite du faisceau d'irradiation.

Sur la Figure 12, on a représenté un objet-test 100 selon le deuxième mode de réalisation , disposé sur un film 106, présentant une face 107a sur laquelle est disposé l'objet-test 100, et une face 107b destinée à venir en utilisation sur l'imageur.

Bien que le film 106 soit décrit en liaison avec le deuxième mode de réalisation, le film peut également être utilisé de manière évidente avec l'objet test selon le premier mode de réalisation.

Le film 106 est un film adhésif double face, les deux faces 107a et 107b du film 106 étant adhésives.

Dans une première variante, au moins l'adhésif de la face 107a du film 100 en contact avec la partie de base du corps 102 de l'objet test 100 a un faible pouvoir adhésif et permet de coller et de décoller de nombreuses fois l'objet-test 100 collé sur celle-ci, afin de permettre de repositionner l'objet-test disposé sur cette face 107a. Le film 106 est alors collé sur l'imageur par sa face 107b, l'adhésif de la face 107b pouvant ou non avoir un faible pouvoir adhésif et permettre de repositionner plusieurs fois le film 106 sur l'imageur. On peut alors coller le film 106 sur toute la surface de l'imageur 12, afin de pouvoir positionner un ou des objets-test sur toute la surface de l'imageur.

Dans une deuxième variante, au moins l'adhésif de la face 107b du film 100 en contact avec l'imageur a un faible pouvoir adhésif qui permet de coller et de décoller de nombreuses fois l'objet-test 100 de la surface de l'imageur 12, l'adhésif de la face 107a en contact avec la partie de base du corps 102 de l'objet test 100 pouvant ou non un avoir un faible pouvoir adhésif afin de permettre de repositionner plusieurs fois le film 106 sur l'objet test 100. Dans cette variante, le film 106 est donc solidaire de la face de base du corps 102 de l'objet test 100, alors que dans la variante précédente, le film 106 est solidaire de la surface de l'imageur sur laquelle sont disposés les objets test.

Le film 106 est en matière plastique, très fin, flexible, d'une épaisseur totale de 0,1 mm à 0,15 mm, et reste donc radiotransparent.

On peut en outre prévoir une languette 108 en polyester, placée dans la première variante entre la face de base du corps 102 de l'objet test et la face 107a du film 106, et dans la deuxième variante entre la face 107b du film 106 et la surface de l'imageur 12. La languette 108 est en saillie entre l'au moins une face à faible pouvoir adhésif et soit la surface de base du corps 102 (première variante) soit la surface de l'imageur (deuxième variante) et permet de décoller plus facilement l'objet test pour le repositionner.

On peut ainsi positionner plus facilement les objets-test sur l'imageur, pour des angles de rotation du statif de l'appareil de rayonnement par radiothérapie par exemple de 90° ou 270°, pour lesquels les objets-test doivent être collés sur l'imageur pour ne pas tomber de la surface de l'imageur.

On pourrait également, pour parvenir au même résultat de positionnement/repositionnement aisé de l'objet-test, disposer une peinture aimantée, à la fois sur la surface de base du corps 102 de l'objet-test 100, et sur la surface de l'imageur 100.

## Revendications

1. Objet-test (100) pour un test de contrôle de qualité d'un appareil de traitement par radiothérapie (6), l'objet-test étant constitué d'un corps (102) de densité électronique d2 et d'au moins un élément (105a, 105b) intégré sur une surface du corps (102) ou noyé dans celui-ci et apte à être distingué du corps (102) par imagerie par faisceaux de rayonnements ionisants, le corps (102) comprenant en outre une surface de base sur laquelle il repose en utilisation et des moyens d'alignement (103) sur un bord d'un faisceau lumineux de simulation émis par une source lumineuse située à l'intérieur de l'appareil de traitement par radiothérapie (6), ledit bord du faisceau lumineux simulant un bord d'un faisceau d'irradiation émis par l'appareil de traitement par radiothérapie (6) pour traiter un patient, le corps (102) étant destiné en utilisation à être placé de telle sorte que l'au moins un élément (105a, 105b) se trouve dans le faisceau d'irradiation émis par l'appareil de traitement par radiothérapie, le corps étant un corps mince et lesdits moyens d'alignement (103) étant espacés dudit ou desdits éléments (105a, 105b), de telle sorte que, dans le plan de la surface de base du corps (102), la projection orthogonale des moyens d'alignement (103) et la projection orthogonale dudit ou desdits éléments (105a, 105b) sont espacées d'une distance prédéterminée h, **caractérisé par le fait que** l'objet-test (1) a une forme rectangulaire, plate,
et l'au moins un élément sont au moins deux éléments aptes à être distingués du corps par imagerie (105a, 105b), intégrés chacun à un montant (101a, 101b) en saillie sur la surface du corps (102) opposée à la surface de base, et les moyens d'alignement (103) étant parallèles à un bord longitudinal de l'objet-test (100) et à la ligne joignant les au moins deux éléments (105a, 105b), l'épaisseur du corps (102) de l'objet-test (100) dans sa zone destinée à détecter la limite du faisceau d'irradiation émis par l'appareil de traitement par radiothérapie (6) est inférieure à l'épaisseur du corps (102) de l'objet-test (100) dans sa zone portant les au moins deux éléments (105a, 105b).

2. Objet-test (100) selon la revendication 1, **caractérisé par le fait que** les au moins deux éléments (105a, 105b) ont une densité électronique d1 différente de d2 et que le rapport entre la densité électronique d1 et la densité électronique d2 est supérieur ou égal à 2, de préférence supérieur à 10.

3. Objet-test (100) selon la revendication 2, **caractérisé par le fait que** les au moins deux éléments de densité électronique d1 sont des billes (105a, 105b) partiellement ou totalement noyées dans le corps (102) de densité électronique d2, ou des fils incorporés dans le corps (2 ; 102) de densité électronique d2.

4. Objet-test (100) selon la revendication 3, **caractérisé par le fait que** les billes (105a, 105b) ont un diamètre de 2 mm.

5. Objet-test (100) selon l'une des revendications 1 à 4, **caractérisé par le fait que** le corps (102) de densité électronique d2 est en polymère thermoplastique, et que l'au moins un élément (105a, 105b) de densité électronique d1 est en métal, de préférence en tungstène.

6. Objet-test (100) selon la revendication 1, **caractérisé par le fait que** les au moins deux éléments (105a, 105b) sont des trous traversants, percés dans le corps de densité électronique d2.

7. Objet-test (100) selon l'une des revendications 1 à 6, **caractérisé par le fait que** les moyens d'alignement (103) sur un bord du faisceau lumineux sont constitués par une ligne principale (3) gravée ou imprimée sur au moins une partie d'une surface (2) de l'objet-test (1), la ligne principale (3) ayant de préférence une largeur de 0,2 mm.

8. Objet-test (100) selon la revendication 1, **caractérisé par le fait que** l'objet-test (1) a une épaisseur principale 0,5 mm, que les au moins deux éléments aptes à être distingués du corps par imagerie (105a, 105b) sont des billes de diamètre de 2 mm, intégrées aux montants (101a, 101b), les montants (101a, 101b) ayant de préférence une hauteur à partir de la surface de base de 2 mm.

9. Objet-test, **caractérisé par le fait qu'**il est constitué d'un cadre rectangulaire, chaque bord du cadre portant ou étant constitué par un objet-test (100) selon l'une quelconque des revendications 1 à 8, les aux moins deux éléments (105a, 105b) pour chaque objet-test (100) étant situés à l'intérieur du cadre.

10. Objet-test selon l'une des revendications 1 à 9, **caractérisé par le fait qu'**il comporte en outre un film mince (106) coopérant avec la surface de base du corps (102), au moins une face (107a, 107b) du film (106) permettant un positionnement/repositionnement aisé de l'objet-test sur les moyens de détection des faisceaux d'irradiation de l'appareil de traitement par radiothérapie.

11. Objet-test selon la revendication 10, **caractérisé par le fait que** le film mince (106) est un film adhésif double face, au moins l'une des deux faces (107a,
107b) présentant un faible pouvoir adhésif permettant de positionner/repositionner l'objet test, ou une couche de peinture aimantée.

12. Procédé de contrôle de qualité d'un appareil de traitement par radiothérapie (6), **caractérisé par le fait qu'**il comprend les étapes de :
a) positionnement de quatre objets-test (100), selon l'une quelconque des revendications 1 à 8 et 10 à 11 lorsqu'elles dépendent des revendications 1 à 8, sur des moyens de détection des faisceaux d'irradiation ou sur tout autre support interposé entre ces moyens de détection des faisceaux d'irradiation et le collimateur (12) de l'appareil de traitement par radiothérapie avant ou après alignement des moyens d'alignement (103) des quatre objets-test (100) sur chacun des quatre bords d'un faisceau lumineux de simulation destiné à matérialiser le faisceau d'irradiation émis par l'appareil de traitement par radiothérapie, les éléments (105a, 105b) de chacun des quatre objets-test (100) étant placés à l'intérieur du faisceau lumineux ;
b) imagerie des objets-test (100) par le faisceau d'irradiation détecté à l'aide des moyens de détection des faisceaux d'irradiation ;
c) calcul sur l'image obtenue, pour chaque objet-test (100), de la distance entre les éléments (105a, 105b) et le bord du faisceau d'irradiation ;
d) déduction, pour chaque bord du faisceau d'irradiation, en fonction de la distance prédéterminée h entre les éléments (105a, 105b) et les moyens d'alignement (103) de la distance entre le bord du faisceau d'irradiation du bord du faisceau lumineux de simulation ;
e) facultativement, lorsque chaque objet-test présente deux éléments (105a, 105b), déduction à partir de l'image obtenue de l'orthogonalité et du parallélisme des bords de faisceau lumineux de simulation ou/et du parallélisme des bords de faisceau lumineux par rapport aux bords de faisceau d'irradiation ;
f) correction de la position de la source lumineuse (10), afin de faire correspondre chaque bord du faisceau lumineux avec chaque bord correspondant du faisceau d'irradiation ; et
g) répétition facultative des étapes précédentes jusqu'à ce que la distance entre les bords du faisceau d'irradiation et les bords du faisceau lumineux soit inférieure ou égale à une valeur seuil, et/ou jusqu'à ce que les deux bords adjacents des faisceaux soient perpendiculaires et que les deux bords opposés des faisceaux soient parallèles.

## Patentansprüche

1. Testobjekt (100) für einen Test zur Qualitätskontrolle eines Strahlentherapiegeräts (6), wobei das Testobjekt aus einem Körper (102) mit der Elektronendichte d2 und aus mindestens einem Element (105a, 105b) gebildet ist, das an einer Fläche des Körpers (102) integriert oder darin eingebettet ist und in der Lage ist, mittels Bilderzeugung mit Strahlen ionisierender Strahlung von dem Körper (102) unterschieden zu werden, wobei der Körper (102) ferner eine Grundfläche umfasst, auf der er während des Gebrauchs aufliegt, und Mittel zum Ausrichten (103) nach einem Rand eines Simulationslichtstrahls, der von einer Lichtquelle ausgesendet wird, die sich im Inneren des Strahlentherapiegeräts (6) befindet, wobei der Rand des Lichtstrahls einen Rand eines Bestrahlungsstrahls simuliert, der von dem Strahlentherapiegerät (6) zur Behandlung eines Patienten ausgesendet wird, wobei der Körper (102) während des Gebrauchs so platziert sein soll, dass sich das mindestens eine Element (105a, 105b) in dem Bestrahlungsstrahl befindet, der von dem Strahlentherapiegerät ausgesendet wird, wobei der Körper ein dünner Körper ist und die Ausrichtungsmittel (103) von dem oder den Elementen (105a, 105b) derart beabstandet sind, dass, in der Ebene der Grundfläche des Körpers (102), die Orthogonalprojektion der Ausrichtungsmittel (103) und die Orthogonalprojektion des oder der Elemente (105a, 105b) um einen vorher festgelegten Abstand h beabstandet sind, **dadurch gekennzeichnet, dass** das Testobjekt (1) eine rechteckige, flache Form aufweist und das mindestens eine Element mindestens zwei Elemente sind, die in der Lage sind, von dem Körper durch Bilderzeugung (105a, 105b) unterschieden zu werden und jeweils an einer Stütze (101a, 101b) integriert sind, die auf der Fläche des Körpers (102) vorsteht, die der Grundfläche gegenüberliegt, und wobei die Ausrichtungsmittel (103) parallel zu einer Längskante des Testobjekts (100) und zu der Linie verlaufen, die die mindestens zwei Elemente (105a, 105b) verbindet, wobei die Dicke des Körpers (102) des Testobjekts (100) in dem Bereich, der für die Detektion der Begrenzung des Bestrahlungsstrahls bestimmt ist, der von dem Strahlentherapiegerät (6) ausgesendet wird, geringer ist als die Dicke des Körpers (102) des Testobjekts (100) in dem Bereich, der die mindestens zwei Elemente (105a, 105b) trägt.

2. Testobjekt (100) nach Anspruch 1, **dadurch gekennzeichnet, dass** die mindestens zwei Elemente (105a, 105b) eine Elektronendichte d1 aufweisen, die sich von d2 unterscheidet, und dass das Verhältnis zwischen der Elektronendichte d1 und der Elektronendichte d2 größer oder gleich 2 ist, vorzugsweise größer als 10.

3. Testobjekt (100) nach Anspruch 2, **dadurch gekennzeichnet, dass** die mindestens zwei Elemente mit der Elektronendichte d1 Kügelchen (105a, 105b) sind, die teilweise oder vollständig in den Körper (102) mit der Elektronendichte d2 eingebettet sind, oder Fäden, die in den Körper (2; 102) mit der Elektronendichte d2 eingebracht sind.

4. Testobjekt (100) nach Anspruch 3, **dadurch gekennzeichnet, dass** die Kügelchen (105a, 105b) einen Durchmesser von 2 mm aufweisen.

5. Testobjekt (100) nach einem der Ansprüche 1 bis 4, **dadurch gekennzeichnet, dass** der Körper (102) mit der Elektronendichte d2 aus einem thermoplastischen Polymer gefertigt ist und dass das mindestens eine Element (105a, 105b) mit der Elektronendichte d1 aus Metall, vorzugsweise aus Wolfram, gefertigt ist.

6. Testobjekt (100) nach Anspruch 1, **dadurch gekennzeichnet, dass** die mindestens zwei Elemente (105a, 105b) durchgehende Löcher sind, die in den Körper mit der Elektronendichte d2 gebohrt sind.

7. Testobjekt (100) nach einem der Ansprüche 1 bis 6, **dadurch gekennzeichnet, dass** die Mittel zum Ausrichten (103) nach einem Rand des Lichtstrahls von einer Hauptlinie (3) gebildet sind, die in zumindest einem Teil einer Fläche (2) des Testobjekts (1) eingeritzt oder darauf aufgedruckt ist, wobei die Hauptlinie (3) vorzugsweise eine Breite von 0,2 mm aufweist.

8. Testobjekt (100) nach Anspruch 1, **dadurch gekennzeichnet, dass** das Testobjekt (1) eine grundsätzliche Dicke von 0,5 mm aufweist, dass die mindestens zwei Elemente, die in der Lage sind, von dem Körper mittels Bilderzeugung (105, 105b) unterschieden zu werden, Kügelchen mit einem Durchmesser von 2 mm sind, die an Stützen (101a, 101b) integriert sind, wobei die Stützen (101a, 101b) vorzugsweise eine Höhe ab der Grundfläche von 2 mm aufweisen.

9. Testobjekt, **dadurch gekennzeichnet, dass** es aus einem rechteckigen Rahmen gebildet ist, wobei jede Kante des Rahmens ein Testobjekt (100) nach einem der Ansprüche 1 bis 8 trägt oder davon gebildet ist, wobei sich die mindestens zwei Elemente (105a, 105b) für jedes Testobjekt (100) im Inneren des Rahmens befinden.

10. Testobjekt nach einem der Ansprüche 1 bis 9, **dadurch gekennzeichnet, dass** es ferner eine dünne Schicht (106) aufweist, die mit der Grundfläche des Körpers (102) zusammenwirkt, wobei mindestens eine Seite (107a, 107b) der Schicht (106) eine einfache Positionierung/Neupositionierung des Testobjekts auf den Mitteln zur Detektion der Bestrahlungsstrahlen des Strahlentherapiegeräts ermöglicht.

11. Testobjekt nach Anspruch 10, **dadurch gekennzeichnet, dass** die dünne Schicht (106) eine doppelseitige Klebefolie ist, wobei mindestens eine der zwei Seiten (107a, 107b) eine geringe Klebkraft aufweist, die zulässt, dass das Testobjekt positioniert/neu positioniert wird, oder ein magnetischer Farbanstrich.

12. Verfahren zur Qualitätskontrolle eines Strahlentherapiegeräts (6), **dadurch gekennzeichnet, dass** es folgende Schritte umfasst:
a) Positionieren von vier Testobjekten (100) nach einem der Ansprüche 1 bis 8 und 10 bis 11, wenn sie von Anspruch 1 bis 8 abhängig sind, auf Mitteln zur Detektion der Bestrahlungsstrahlen oder jedem anderen Träger, der zwischen diesen Mitteln zur Detektion der Bestrahlungsstrahlen und dem Kollimator (12) des Strahlentherapiegeräts angeordnet ist, vor oder nach dem Ausrichten der Ausrichtungsmittel (103) der vier Testobjekte (100) nach jedem der vier Ränder eines Simulationslichtstrahls, der den Bestrahlungsstrahl sichtbar machen soll, der von dem Strahlentherapiegerät ausgesendet wird, wobei die Elemente (105a, 105b) von jedem der vier Testobjekte (100) im Inneren des Lichtstrahls platziert werden;
b) Erzeugen eines Bilds der Testobjekte (100) mit dem Bestrahlungsstrahl, der mithilfe der Mittel zur Detektion der Bestrahlungsstrahlen detektiert wird;
c) für jedes Testobjekt (100) Berechnen des Abstands zwischen den Elementen (105a, 105b) und dem Rand des Bestrahlungsstrahls in dem erhaltenen Bild;
d) für jeden Rand des Bestrahlungsstrahls Ableiten des Abstands zwischen dem Rand des Bestrahlungsstrahls von dem Rand des Simulationslichtstrahls in Abhängigkeit von dem vorher festgelegten Abstand h zwischen den Elementen (105a, 105b) und den Ausrichtungsmitteln (103);
e) wenn jedes Testobjekt zwei Elemente (105a, 105b) aufweist, wahlweise Ableiten der Orthogonalität und der Parallelität der Ränder des Simulationslichtstrahls und/oder der Parallelität der Ränder des Lichtstrahls bezogen auf die Ränder des Bestrahlungsstrahls aus dem erhaltenen Bild;
f) Korrigieren der Position der Lichtquelle (10), um jeden Rand des Lichtstrahls mit jedem entsprechenden Rand des Bestrahlungsstrahls in Übereinstimmung zu bringen; und
g) wahlfreies Wiederholen der vorhergehenden Schritte, bis der Abstand zwischen den Rändern des Bestrahlungsstrahls und den Rändern des Lichtstrahls geringer als ein oder gleich einem Schwellwert ist, und/oder bis die beiden benachbarten Ränder der Strahlen senkrecht verlaufen und die beiden gegenüberliegenden Ränder der Strahlen parallel verlaufen.

## Claims

1. A test object (100) for a quality control test of a radiotherapy treatment apparatus (6), the test object being constituted by a body (102) with an electronic density d2 and at least one element (105a, 105b) integrated onto a surface of the body (102) or embedded therein and capable of being distinguished from the body (102) by imaging by ionizing radiation beams, the body (102) further comprising a base surface on which it rests in use and alignment means (103) on an edge of a simulation light beam emitted by a light source located inside the radiotherapy treatment apparatus (6), said light beam edge simulating an edge of a radiation beam emitted by the radiotherapy treatment apparatus (6) for treating a patient, the body (102) being designed, in use, to be arranged such that the at least one element (105a, 105b) is in the radiation beam emitted by the radiotherapy treatment apparatus, the body being a thin body and said alignment means (103) being spaced apart from said one or more elements (105a, 105b) such that, in the plane of the base surface of the body (102), the orthogonal projection of the alignment means (103) and the orthogonal projection of said one or more elements (105a, 105b) are spaced apart by a predetermined distance h, **characterized by** the fact that the test object (1) has a flat, rectangular shape and the at least one element is at least two elements capable of being distinguished from the body by imaging (105a, 105b), each integrated to a post (101a, 101b) protruding from the surface of the body (102) opposite to the base surface, and the alignment means (103) being parallel to a longitudinal edge of the test object (100) and to the line joining the at least two elements (105a, 105b), the thickness of the body (102) of the test object (100) in its area designed to detect the limit of the radiation beam emitted by the radiotherapy treatment apparatus (6) is lower than the thickness of the body (102) of the test object (100) in its area carrying the at least two elements (105a, 105b).

2. The test object (100) according to claim 1, **characterized by** the fact that the at least two elements (105a, 105b) have an electronic density d1 different from d2 and that the ratio between the electronic density d1 and the electronic density d2 is greater than or equal to 2, preferably greater than 10.

3. The test object (100) according to claim 2, **characterized by** the fact that the at least two elements with an electronic density d1 are balls (105a, 105b), partially or completely embedded in the body (102) with an electronic density d2, or wires incorporated in the body (2; 102) with an electronic density d2.

4. The test object (100) according to claim 3, **characterized by** the fact that the balls (105a, 105b) have a diameter of 2 mm.

5. The test object (100) according to one of claims 1 to 4, **characterized by** the fact that the body (102) with an electronic density d2 is made of a thermoplastic polymer, and by the fact that the at least one element (105a, 105b) with an electronic density d1 is made of a metal, preferably made of tungsten.

6. The test object (100) according to claim 1, **characterized by** the fact that the at least two elements (105a, 105b) are through holes, drilled in the body with an electronic density d2.

7. The test object (100) according to one of claims 1 to 6, **characterized by** the fact that the alignment means (103) on an light beam edge are constituted by a main line (3) etched or printed on at least a portion of a surface (2) of the test object (1), the main line (3) preferably having a width of 0,2 mm.

8. The test object (100) according to claim 1, **characterized by** the fact that the test object (1) has a main thickness of 0,5 mm, and by the fact that the at least two elements capable of being distinguished from the body by imaging (105a, 105b) are balls with a diameter of 2 mm, integrated to the posts (101a, 101b), the posts (101a, 101b) preferably having a height from the base surface of 2 mm.

9. A test object **characterized by** the fact that it is made of a rectangular frame, each edge of the frame carrying or being constituted by a test object (100) according to any one of claims 1 to 8, the at least two elements (105a, 105b) for each test object (100) being arranged within the frame.

10. The test object according to one of claims 1 to 9, **characterized by** the fact that it further comprises a thin film (106) cooperating with the base surface of the body (102), at least one face (107a, 107b) of the film (106) allowing to easily position/reposition the test object on the means for detecting radiation beams of the radiotherapy treatment apparatus.

11. The test object according to claim 10, **characterized by** the fact that the thin film (106) is a double-sided adhesive film, at least one of the two faces (107a, 107b) exhibiting a low tackiness allowing to position/reposition the test object, or a layer of magnetic paint.

12. A quality control method of a radiotherapy treatment apparatus (6), **characterized by** the fact that it comprises the following steps:
a) positioning four test objects (100), according to any one of claims 1 to 8 and 10 to 11 when they depend on claims 1 to 8, on means for detecting radiation beams or on any other support interposed between these means for detecting radiation beams and the collimator (12) of the radiotherapy treatment apparatus before or after aligning the alignment means (103) of the four test objects (100) on each of the four edges of a simulation light beam designed to materialize the radiation beam emitted by the radiotherapy treatment apparatus, the elements (105a, 105b) of each of the four test objects (100) being arranged within the light beam;
b) imaging the test objects (100) by the radiation beam detected using the means for detecting radiation beams;
c) calculating, on the obtained image, for each test object (100), the distance between the elements (105a, 105b) and the radiation beam edge;
d) deducing, for each radiation beam edge, depending on the predetermined distance h between the elements (105a, 105b) and the alignment means (103), the distance between the radiation beam edge and the simulation light beam edge;
e) optionally, when each test object has two elements (105a, 105b), deducing from the image obtained the orthogonality and the parallelism of the simulation light beam edges and/or the parallelism of the light beam edges with respect to the radiation beam edges;
f) correcting the position of the light source (10), so as to match each light beam edge with each corresponding radiation beam edge; and
g) optionally, repeating the previous steps until the distance between the radiation beam edges and the light beam edges is lower than or equal to a threshold value and/or until two adjacent beam edges are perpendicular and two opposite beam edges are parallel.
